# EUROPEAN PATENT SPECIFICATION

(11) **EP 2 694 097 B1**
(45) Date of publication and mention of the grant of the patent: **13.07.2016**
(21) Application number: 12710874.4
(22) Date of filing: 20.03.2012
(51) Int. Cl.: A61K 38/34, A61P 13/12, A61P 29/00

(54) **PEPTIDES FOR SUPPRESSING INFLAMMATION REACTIONS IN HEMODIALYSIS**
PEPTIDE ZUR UNTERDRÜCKUNG VON ENTZÜNDUNGSREAKTIONEN BEI DER HÄMODIALYSE
PEPTIDES PERMETTANT DE SUPPRIMER LES RÉACTIONS D'INFLAMMATION DANS UNE HÉMODIALYSE

(30) Priority: 07.04.2011 EP 11002917; 07.04.2011 US 201161457479 P
(43) Date of publication of application: 12.02.2014
(73) Proprietor: Fresenius Medical Care Deutschland GmbH, 61352 Bad Homburg (DE)
(72) Inventor: PASSLICK-DEETJEN, Jutta, 35392 Giessen (DE); FISLAGE, Rainer, 66606 St. Wendel (DE); CATANIA, Anna, I-20122 Milano (IT); GATTI, Stefano, I-22100 Como (IT); KUHN, Anja, 80995 Munich (DE); STEPPAN, Sonja, 63263 Neu-Isenburg (DE)
(74) Representative: Breuninger, Marcus
(86) International application number: PCT/EP2012/001227
(87) International publication number: WO 2012/136312

(56) References cited:
- WO-A1-03/094990
- WO-A1-2010/129248
- WO-A2-2004/104019
- US-A1- 2010 143 438
- KELLY ET AL: "Immobilized alpha-melanocyte stimulating hormone 10-13 (GKPV) inhibits tumor necrosis factor-alpha stimulated NF-kappaB activity", PEPTIDES, ELSEVIER, AMSTERDAM, vol. 27, no. 2, 1 February 2006 (2006-02-01), pages 431-437, XP005269001, ISSN: 0196-9781, DOI: DOI:10.1016/J.PEPTIDES.2005.03.062
- GATTI S ET AL: "Inhibitory Effects of the Peptide (CKPV)2 on Endotoxin-Induced Host Reactions", JOURNAL OF SURGICAL RESEARCH, ACADEMIC PRESS INC., SAN DIEGO, CA, US, vol. 131, no. 2, 1 April 2006 (2006-04-01) , pages 209-214, XP024952797, ISSN: 0022-4804, DOI: DOI:10.1016/J.JSS.2005.08.009 [retrieved on 2006-04-01]

## Description

### Technical Field

The invention relates to a conjugate L-P comprising a linker L and a peptide P. The conjugate L-P can be employed as an intermediate in the preparation of the construct S-L-P comprising a surface S of a solid support. Further, the conjugate L-P is useful in hemodialysis, particularly for the prevention and/or suppression of inflammatory responses and processes.

### Background for the Invention

Dialysis is a method of cleansing an individual's blood when the kidneys fail to function properly. Dialysis eliminates the body's extra salt, wastes and water, and assists to control blood pressure.

Two types of dialysis are usually distinguished: hemodialysis and peritoneal dialysis. In hemodialysis, a patient is connected via tubes to an artificial kidney. Blood is pumped out of the body to the artificial kidney which filters the blood, and then the blood is returned to the body. In peritoneal dialysis, the inside lining of the patient's peritoneal cavity is used as a natural filter. Wastes are removed by means of a fluid, the dialysate, which is washed in and out of the peritoneal cavity in cycles.

In hemodialysis, an artificial kidney (hemodialyzer) is used to remove waste, extra chemicals and excess fluid from the blood. To transfer a dialysis patient's blood into the artificial kidney, vascular access is required. A common access is made by joining an artery to a vein under the skin and thus creating a fistula. The fistula is then penetrated, allowing access to blood. Occasionally, an access is made by means of a catheter, which is inserted into a large vein in the neck. This type of access may be temporary, but in many instances may be used for long-term treatment. As with any vascular access or catheter treatment, the catheter creates a direct communication between a patient's aseptic internal environment and the outside world. Common complications of this communication are infections, inflammations and thrombotic or infiltrative blockage of the access.

Inflammation represents a major medical complication associated with dialysis. In fact, although the benefits of hemodialysis and peritoneal dialysis are numerous, many of the complications associated with each may be life threatening. Each technique has its own or similar complications including, but not limited to, ultra filtration failure, compliance, obesity, clearance, and poor fluid compliance.

Inflammation is a major factor for high mortality in patients with end stage renal disease (ESRD). It is considered that inflammation plays a primary role in arterial damage in dialysis patients. Although the precise mechanisms leading to this inflammatory state in ESRD are unclear, low-grade infection, repeated exposure to dialysis filters and auto-oxidation products are discussed as likely inciting factors in these patients (cf. C. Zocalli et al., Blood purification, 2003, 21, 29-36; and P. Stenvinkel et al., Semin. Dial., 2002, 15, 329-37).

There is a demand for methods that cause less inflammation in hemodialysis patients.

a-MSH (melanocyte-stimulation hormone) derives from a precursor hormone, namely propiomelanocortin (POMC). The post-translational processing of POMC yields in peptide hormones such as ACTH, a-MSH - g-MSH and b-endorphin.

a-MSH has multiple effects on the host.

The stimulatory effect of a-MSH in pigment cells has been known for over 50 years. More recent findings indicate that a-MSH controls food-intake and exocrine secretions, modulates fever (cf. D. B. Richards et al., Peptides 1984, 5(4), 815-7) and inflammatory reactions and has an antimicrobial effect (cf. e.g. A. Catania et al., J Leukoc Biol. 2000, 67(2), 233-9). a-MSH induces Fos expression in supraoptic oxytocin neurons (N. Sabatier et al., J. Neuroscience, 2003, 23(32), 10351-8).

[Nle4-d-Phe7] a-MSH (NDP-MSH) has been described as a protease-stable a-MSH analogue (cf. T. K. Sawyer et al., Proc. Natl. Acad. Sci. USA, 1980, 77(10), 5754-58).

Ac-Lys-Pro-Val-NH₂ (KPV), the terminal tripeptide of a-MSH, which is N-acetylated and C-amidated has shown to exhibit almost the same anti-inflammatory effect as full-length a-MSH.

WO 88/00833 discloses an antipyretic tripeptide, having the amino acid sequence lysine-proline-valine, and a method for utilizing the tripetide to reduce fever and inflammation in mammals.

US 2009/0069242 describes peptide analogues of a-MSH, which possess an increased efficacy compared to the native a-MSH peptide.

US 2005/0130901 and US 2006/0122121 relate to peptides with antimicrobial activity. The peptides are octomeric peptides modified from a-MSH.

US 2010/143438 and WO 2010/129248 disclose S-L-alphaMSH constructs for use in treating inflammation.

WO 2004/004551 discloses a composition and method for controlling host response to organ and/or tissue transplantation and grafting. a-MSH protects organ and tissue after transplantation by controlling factors within the donor, host and of the organ or tissue to be transplanted.

WO 00/56353 relates to an uro-genital condition system comprising a carrier, at least one polypeptide including an amino acid sequence of KPV or a biologically functional equivalent thereof; wherein the carrier carries the polypeptide. The polypeptide may include a dimer formed from the amino acid sequence.

J. M. Kelly et al., Peptides, 2006, 27, 431-7 discloses that immobilized a-melanocyte stimulating hormone 10-13 (GKPV) inhibits tumor necrosis factor-α stimulated NF-kB activity.

The capacity of the dimer (CKPV)2 to inhibit endotoxin-induced host reactions suggests that it may be useful in treatment of inflammatory disorders (S. Gatti et al., J Surg Res, 2006, 131(2), 209-14).

Synthesis and activity of the dimer [Ac-CKPV]₂ are also known (cf. A. Catania et al., J Pept Res., 2005, 66(1), 19-26).

It is known that a-MSH concentrations are elevated in patients on chronic hemodialysis. Concentrations of up to 30 ng/l are observed whereas healthy patients show concentrations of up to 25 ng/l only. The concentration of a-MSH is elevated particularly when simultaneously an elevated concentration of endotoxin can be observed. Possibly, a-MSH release is induced by cytokines in order to limit their effect (cf. L. Airaghi et al., Nephrol Dial Transplant, 2000, 15, 1212-6).

Anti-inflammatory effects of a-MSH-related tripeptides *in vitro* and *in vivo* were also investigated (Broska et al., Endocrine reviews, 2008, 29(5), 581-602).

WO 03/094990 relates to oligo- and polysaccharides containing the sugar structural element N-acylglucosamine or N-acylgalactosamine, in addition to the use thereof for producing hemocompatible surfaces and methods for coating surfaces with said oligo- and polysaccharides, which constitute the common biosynthetic precursor substances of heparin, heparan sulfates and chitosan.

US 2005/074485 and WO 2004/104019 disclose a composition and method of treatment in which anti-microbial and anti-inflammatory peptides, preferably KPV or a dimer thereof, are used for hemodialysis and peritoneal dialysis in dialysate and gel. The anti-microbial and anti-inflammatory peptides may be used in peritoneal dialysis to increase diuresis. Other embodiments include use of the anti-microbial and anti-inflammatory peptides in locking solutions used for catheters and other vascular access tubing or peritoneal access tubing.

These compositions, however, are not satisfactory in every respect and there is a demand for further improvements of hemodialysis.

### Brief Description of the Figures

Figures 1A and 1 B summarize the results of the pre-stimulation experiments.
Figures 2A and 2B summarize the results of the co-incubation experiments.
Figure 3 summarizes the effect of heat treatment of KPV on the inhibition of TNF-α.
Figure 4 summarizes the effect of heat treatment of KPV on the inhibition of IL-6.
Figure 5 summarizes the results of the inhibition of TNF-α at different concentrations of KPV.

### Detailed Description of the Invention

It is an object of the invention to provide improvements for hemodialysis and other methods where body fluids are guided through extracorporeal circuits, particularly with respect to the occurrence of inflammation.

This object is achieved by the subject-matter of the patent claims.

It has been surprisingly found that surfaces that are modified with small peptides having the C-terminus of amidated and acylated KPV are particularly useful in hemodialysis with respect to the prevention and/or suppression of inflammation.

It has been found that the invention may also be worked with complete aMSH or fractions thereof. For the purpose of this invention, peptides having the C-terminus of amidated and acylated KPV may also be replaced by aMSH or less preferred with other fractions thereof. Fractions may be peptides comprising an amino acid sequence of at least 3 amino acids which is also comprised in aMSH.

Compared to a-MSH said peptides have several advantages. They are small and inexpensive molecules that are suitable for large-scale pharmaceutical production. It seems that they do not show a pronounced melanogenic effect, show less adverse side-effects and because of their molecular size, are advantageous for local therapy of several inflammatory disorders.

Compared to conventional immunosuppressive agents, they lower the risk to infections due to their combined anti-inflammatory and antimicrobial efficacy.

The surface S of the solid support is not particularly limited, as long as it is capable for immobilization of the peptide P through the linker L.
Preferably, the surface S is adapted for being contacted with a body fluid, such as blood, serum and plasma.
In a particularly preferred embodiment, the surface S is hemocompatible.
For the purpose of the specification hemocompatibility is preferably defined according to ISO 10993-4 relating to tests concerning the effects of blood contacting the product or compounds on blood or blood components, directly or indirectly during routine use.

Hemocompatibility is preferably achieved by providing the solid support with an external coating, e.g., a hemocompatible layer. The hemocompatible layer can be added directly onto the surface of a preferably non hemocompatible solid support or deposited onto other biostable and/or biodegradable layers. Additional biostable and/or biodegradable and/or hemocompatible layers can be present on top of the hemocompatible layer.
Hemocompatible materials are known to the person skilled in the art. Preferably, the surface S of the solid support is rendered hemocompatible *inter alia* by the presence of the peptide P that is immobilized on the surface S through linker L.

It is preferred that at least one biostable layer is present under the hemocompatible layer. In addition the hemocompatible layer can be coated totally and/or partially with at least one more, above lying biostable and/or biodegradable layer.

Examples of biodegradable substances for the biodegradable layer(s) include polyvalerolactones, polylactonic acid, poly-[epsilon]-decalactones, polyglycolic acid, polylactides, polyglycolides, copolymers of the polylactides and polyglycolides, poly-[epsilon]-caprolactone, polyhydroxybutanoic acid, polyhydroxybutyrates, polyhydroxyvalerates, polyhydroxybutyrate-co-valerates, poly(1,4-dioxane-2,3-diones), poly(1,3-dioxane-2-one), poly-para-dioxanones, polyanhydrides such as polymaleic anhydrides, polyhydroxymethacrylates, fibrin, polycyanoacrylates, polycaprolactonedimethylacrylates, poly-b-maleic acid, polycaprolactonebutyl-acrylates, multiblock polymers such as e.g. from oligocaprolactonedioles and oligodioxanonedioles, polyether ester multiblock polymers such as e.g. PEG and poly(butyleneterephtalates), polypivotolactones, polyglycolic acid trimethyl-carbonates, polycaprolactone-glycolides, poly(g-ethylglutamate), poly(DTH-iminocarbonate), poly(DTE-co-DT-carbonate), poly(bisphenol-A-iminocarbonate), polyiminocarbonates, polyorthoesters, polyglycolic acid trimethyl-carbonates, polytrimethylcarbonates, poly(N-vinyl)-pyrrolidone, polyvinylalcoholes, polyesteramides, polyphosphoesters, glycolated polyesters, polyphosphazenes, poly[p-carboxyphenoxy)propane], polyhydroxypentanoic acid, polyanhydrides, poly(ethyleneoxide-propyleneoxide), soft polyurethanes, polyurethanes with amino acid residues in the backbone, polyether esters such as polyethyleneoxide, polyalkeneoxalates, polyorthoesters as well as their copolymers, carrageenans, lipides, fibrinogen, starch, collagen, protein based polymers, polyamino acids, synthetic polyamino acids, zein, modified zein, polyhydroxyalkanoates, actinic acid, pectic acid, modified and non modified fibrin and casein, carboxymethylsulphate, albumin, moreover hyaluronic acid, chitosan and its derivatives, heparansulphates and its derivatives, heparins, chondroitinsulphate, dextran, b-cyclodextrins, copolymers with PEG and polypropyleneglycol, gummi arabicum, guar, gelatine, collagen, collagen-N-hydroxysuccinimide, lipids, phospholipids, modifications and copolymers and/or mixtures of the afore mentioned substances.

Examples of biostable substances for the biostable layer(s) include polyacrylic acid and polyacrylates as polymethylmethacrylate, polybutylmethacrylate, polyacrylamide, polyacrylonitriles, polyamides, polyetheramides, polyethylenamine, polyimides, polycarbonates, polycarbourethanes, polyvinylketones, polyvinylhalogenides, polyvinylidenhalogenides, polyvinylethers, polyisobutylenes, polyvinylaromates, polyvinylesters, polyvinylpyrollidones, polyoxymethylenes, polytetramethyleneoxide, polyethylene, polypropylene, polytetrafluoroethylene, polyurethanes, polyetherurethanes, silicone-polyetherurethanes, silicone-polyurethanes, silicone-polycarbonate-urethanes, polyolefine elastomeres, polyisobutylenes, EPDM gums, fluorosilicones, carboxymethylchitosanes, polyaryletheretherketones, polyetheretherketones, polyethylenterephthalate, polyvalerates, carboxymethylcellulose, cellulose, rayon, rayontriacetates, cellulosenitrates, celluloseacetates, hydroxyethylcellulose, cellulosebutyrates, cellulose-acetatebutyrates, ethylvinylacetate copolymers, polysulphones, epoxy resins, ABS resins, EPDM gums, silicones as polysiloxanes, polydimethylsiloxanes, polyvinylhalogenes and copolymers, celluloseethers, cellulosetriacetates, chitosanes and copolymers and/or mixtures of these substances.

In a preferred embodiment, the surface S contains at least an area with a surface concentration of the protein P of at least 1.0 nmol/cm², more preferably at least 2.0 nmol/cm², still more preferably at least 5 nmol/cm², yet more preferably at least 10 nmol/cm², most preferably at least 25 nmol/cm² and in particular at least 50 nmol/cm². Preferably, said area is at least 1.0 cm² large.

The surface S is the surface of a solid support. The solid support is not particularly limited. Preferably, the solid support is a medical device, e.g. component of an extracorporeal circuit, preferably of a system or component thereof adapted for hemodialysis, hemofiltration, plasmapheresis, apheresis, extracorporeal membrane oxygenation or assisted blood circulation.

In a preferred embodiment the surface S of the solid support is suitable for the short-or the long-term contact with blood or blood products. Examples of solid supports include medical devices such as prostheses, organs, vessels, aortas, heart valves, tubes, organ spare parts, implants, fibers, hollow fibers, stents, hollow needles, syringes, membranes, tinned goods, blood containers, titrimetric plates, pacemakers, adsorbing media, chromatography media, chromatography columns, dialyzers, connexion parts, sensors, valves, centrifugal chambers, recuperators, endoscopes, filters, and pump chambers. Dialyzers are particularly preferred, especially their components, particularly dialysis membranes.

The solid support may be composed of inorganic and/or organic material, metal or polymers. Suitable polymers may be thermoplastic and are known to the skilled artisan.

The solid support and the surface S, respectively, can assume any shape, e.g. plain, convex and concave and the like. When the solid support is a dialysis membrane, it may be, e.g., fibrous, tissue-like, a hollow fiber, or a flat membrane.
Linkers L that are suitable to immobilize a peptide P on a suitable surface S are known to the skilled person. In this regard it can be referred e.g. to G.T. Hermanson, Bioconjugate Techniques, Second Edition, Academic Press 2008; F. Zaragoza Dörwald, Organic Synthesis on Solid Phase: Supports, Linkers, Reactions, Wiley-VCH; 2 edition 2002; J. M. Guisan, Immobilization Of Enzymes And Cells (Methods in Biotechnology), Humana Press; 2 edition 2006; J.A. Camarero, Biopolymers. 2008, 90(3), 450-8.

The chemical nature of the linker L is not particularly limited. Typical linkers L comprise at least one distal end that is faced to the peptide P and at least one proximal end that is faced to the surface S.

The linker L may have any end-to-end distance from the distal end to the proximal end that is suitable in order to immobilize the peptide P on the surface S of the solid support. Typical (average) lengths are in the range of from a few nanometers to several nanometers or more.

Preferably, the linker L is an organic molecule. It may contain an uninterrupted carbon-carbon chain between the distal end and the proximal end. Preferably, however, in between at least some of the carbon atoms of the chain are heteroatoms which are preferably independently selected from Si, N, S and O.
Preferably, linker L is substantially linear. Preferably, linker L comprises a moiety that is composed of repetition units, like in a polymer or oligomer. For example, when linker L comprises a polyethylene glycol moiety, the repetition units are - (O-CH₂CH₂)ₓ-.

The linker L may be derived from biomolecules, such as peptides, or synthetic, such as polyalkylene glycols, e.g., polyethylene glycols or polypropylene glycols.

The linker L may be composed of several segments that differ from one another in chemical nature but are covalently linked with one another. For example, a first segment of linker L may be peptidic and a second segment of linker L that is covalently bound to said first segment may be a polyalkylene glycol or a polyalkylene oxide.

Preferably, the linker L comprises a poly(alkylene glycol) a poly(alkyleneoxide) segment or moiety.

In a preferred embodiment the linker L does not include any a-amino acid residue, i.e., the linker is non-peptidic.

In a preferred embodiment, the overall molecular weight of the linker L is within the range of from 500 g/mol to 2,000,000 g/mol, more preferably 750 g/mol to 1,000,000 g/mol, still more preferably 1000 g/mol to 500,000 g/mol, yet more preferably 1250 g/mol to 100,000 g/mol, most preferably 1500 g/mol to 50,000 g/mol and in particular 2000 g/mol to 10,000 g/mol.

In a preferred embodiment, the surface S comprises free carboxyl groups (-COOH) which are activated by the use of EDC or other activation compounds. Subsequently, this activated surface S is preferably brought in contact with the peg-modified peptide P to yield the corresponding covalent bond.

The linker L is preferably covalently bound to the surface S, e.g. via an ester bond, ether bond, amide bond, thioether bond, and the like. Chemo selective immobilizations may be achieved, e.g., by amino group to aldehyde linkage, aminooxyacetyl group to glyoxylyl linkage, cysteine residue to thioester linkage, cyclopentadiene residue to benzoquinone linkage, and the like.
Solid supports bearing at their surfaces S reactive functional groups that are capable of reacting with a compatible functional group at the proximal end of liker L are commercially available. For example, the solid supports may bear at their surfaces S reactive functional groups selected from the group consisting of amines, thiols, aldehydes, carboxylic acids, aminooxyacetyl groups, glyoxylyl groups, thioester groups, cyclopentadiene groups, benzoquinones, trialkoxysilanes, and the like.

Methods for introducing said reactive functional groups to the surfaces S of solid supports are also known to the person skilled in the art.

Preferably, the linker L and the surface S of the solid support are linked with one another only by covalent bonds so that between peptide P and surface S there is a continuous chain of covalent bonds, which chain may be linear or branched.
It is also possible that the linker L is non-covalently bound to the surface S, e.g. by biotin-(strept)avidin interaction and the like. Under these circumstances the solid support bears at its surface S, e.g., biotin molecules that are attached to the surface S by conventional methods. The proximal end of the linker L in turn bears a compatible (strept)avidine group so that a molecular complex can be formed thereby immobilizing the linker L at the surface S. A skilled person recognizes that the location of the biotine and the (strept)avidine at surface S and linker L can be exchanged. Biotin-labeling can be easily achieved, e.g., by biotin-pegylation.

Peptide P is composed of proteinogenic amino acids which independently of one another can have D- or L-configuration. Preferably, peptide P consists of a-amino acid residues that have L-configuration.

In a preferred embodiment, peptide P includes not more than 4 a-amino acid residues directly bound to one another through peptide bonds.

In another preferred embodiment, peptide P includes two subsequences each including not more than 4 a-amino acid residues directly bound to one another through peptide bonds, wherein said two subsequences are bound to one another through a non-peptide bond, preferably through a cystin -S-S- bond of two cysteins.

The peptide P may be prepared by solid-phase peptide synthesis and purified by reversed-phase high performance liquid chromatography.

The linker L is preferably covalently bound to the peptide P, e.g. via an ester bond, ether bond, amide bond, thioether bond, and the like. Such a linkage may be achieved, e.g., by amino group to aldehyde linkage, cysteine residue to thioester linkage, amide linkage and the like.

Linkers L bearing at their distal ends reactive functional groups that are capable of reacting with a compatible functional group at the peptide P are commercially available. For example, the linkers L may bear at their distal ends reactive functional groups selected from the group consisting of amines, thiols, alcohols, aldehydes, carboxylic acids, aminooxyacetyl groups, glyoxylyl groups, thioester groups and the like.

It is also possible that the linker L is non-covalently bound to the peptide P, e.g. by biotin-(strept)avidin interaction and the like. Under these circumstances the linker L bears at its distal end, e.g., biotin molecules that are attached to the linker L by conventional methods. The peptide P in turn bears a compatible (strept)avidine group so that a molecular complex can be formed thereby attaching the peptide P to the distal end of the linker L. A skilled person recognizes that the location of the biotine and the (strept)avidine at peptide P and linker L can be exchanged.

Preferably, every linker L bears one peptide P. It is also possible, however, that more than one peptide P is linked to a single linker L. This can be achieved, for example, by means of branched linkers L that bear one proximal end attached to the surface S of the solid support and more than one distal end, e.g. 2, 3 or 4 distal ends, linked to one peptide P each.

One aspect of the invention relates to a conjugate L-P comprising a linker L as defined above covalently bound to a peptide P as defined above.

Preferably, linker L comprises a polyalkylene glycol moiety, particularly preferred a polyethylene glycol moiety (peg) such that the conjugate L-P preferably can be regarded as a peptide P as defined above that is pegylated by the polyethylene glycol moiety of linker L.

In a preferred embodiment, the conjugate L-P according to the invention has a structure according to general formula (VI): 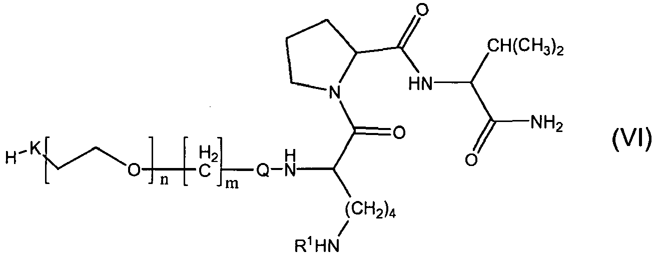 wherein
R¹ is -H or -C(=O)-C₁₋₈-aliphate, preferably acetyl;
Q is a bond or a functional group selected from -C(=O)-, -C(=S)-, -C(=NH)-, - NH-C(=O)-, -NH-C(=S)- and -NH-C(=NH)-;
K is a bond or a functional group selected from -C₁₋₈-alkylene-, -NH-, -O-, -S-, -C(=O)-, or -C(=O)O-;
n is an integer of from 0 to 250, preferably 1 to 25, more preferably 2 to 10; and
m is an integer of from 0 to 12, preferably 1, 2 or 3.

For the purpose of the specification, bivalent functional groups that have two possibilities of being incorporated in a general formula in two different directions, e.g. -NH-C(=O)-, can be incorporated in both directions alternatively.

In a particularly preferred embodiment, R¹ is acetyl, Q is -C(=O)-, m is 2, n is 3 and K is -NH-, so that the conjugate L-P has the following structure:

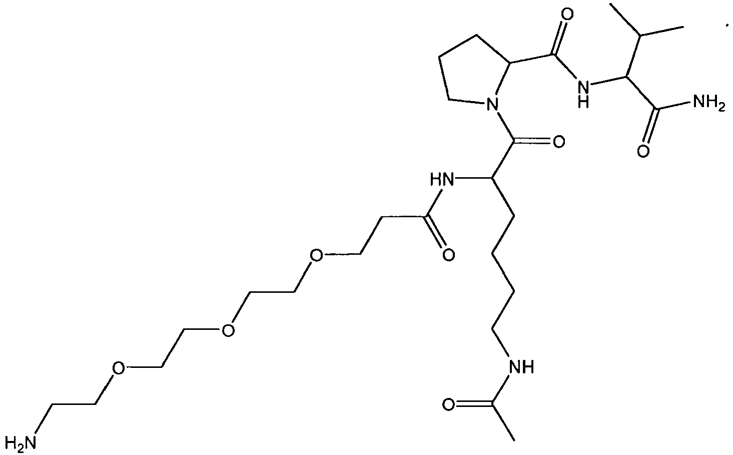

In another preferred embodiment, the conjugate L-P according to the invention has a structure according to general formula (VII): 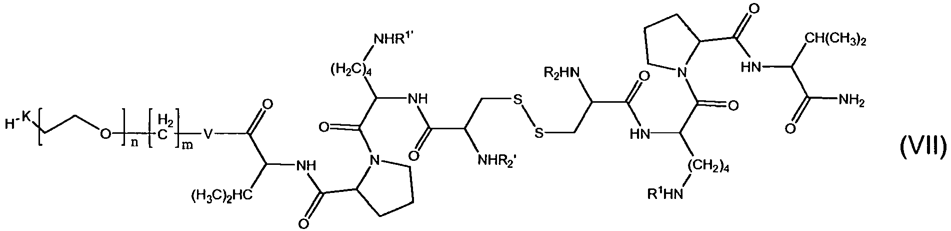 wherein
R¹, R¹', R² and R²' are independently of each other -H or -C(=O)-C₁₋₈-aliphate, preferably acetyl;
V is a bond or a functional group selected from -O- and -NH-, preferably -NH;
K is a bond or a functional group selected from -C₁₋₈-alkylene-, -NH-, -O-, -S-, -C(=O)-, or -C(=O)O- ;
n is an integer of from 0 to 250, preferably 1 to 25, more preferably 2 to 10; and
m is an integer of from 0 to 12, preferably 1, 2 or 3.

In a particularly preferred embodiment, R¹, R¹', R² and R^{2'} are acetyls, V is -NH-, m is 2, n is 1 and K is -NH-, so that the conjugate L-P has the following structure:

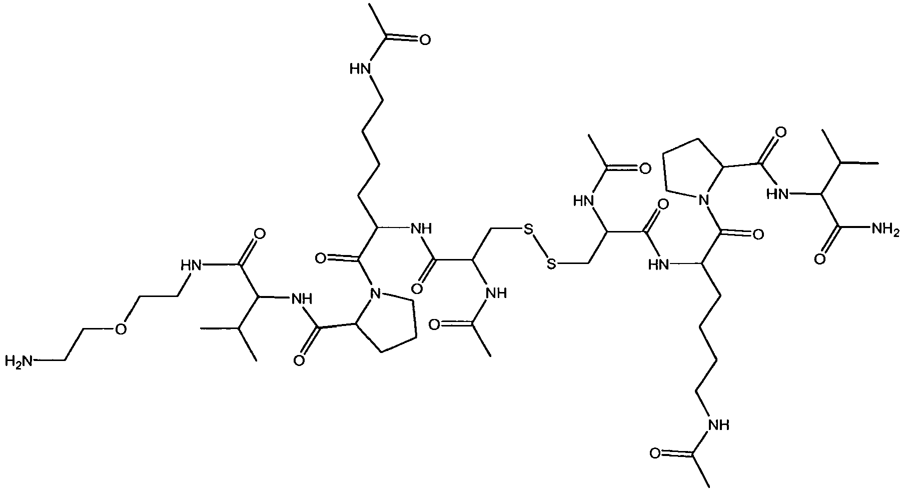

The conjugate L-P according to the invention may be employed as an intermediate in the preparation of the construct S-L-P according to the invention by attaching the conjugate L-P to the surface S of the solid support is appropriate methods (see below).

Alternatively, however, the conjugate L-P according to the invention can be used as medicament as such.

Therefore, a further aspect of the invention relates to a pharmaceutical composition comprising the conjugate L-P according to the invention and a physiologically acceptable carrier.

The pharmaceutical composition according to the invention can be solid, pasty or liquid. It may contain the conjugate L-P within the range of from 0.001 to 99.999 wt.-%, based on the total weight of the composition.

In a preferred embodiment, the pharmaceutical composition is formulated for parenteral administration. Parenteral administration can be carried out, for example, subcutaneously, intravenously, intramuscularly, intraarterially, intraperitoneally, intracutaneously, intraarticularly, intrathecally, intracardially, intravitreally, retrobulbarly, intrapulmonarily and intraosseously.

When administration is parenteral, injectable pharmaceuticals may be prepared in conventional forms, as aqueous or non-aqueous solutions or suspensions; as solid forms suitable for solution or suspension in liquid prior to injection; or as emulsions. Examples of non-aqueous solvents are propylene glycol, polyethylene glycol, vegetable oils such as olive oil, and injectable organic esters such as ethyl oleate. Other suitable excipients are water, saline, dextrose, mannitol, lactose, lecithin, albumin, sodium glutamate, cystine hydrochloride, or the like. In addition, the injectable pharmaceutical compositions may contain minor amounts of non-toxic auxiliary substances, such as wetting agents, pH buffering agents, and the like. If desired, absorption-enhancing preparations (e. g., liposomes) may be used.

In another preferred embodiment, the pharmaceutical composition is formulated for administration via an extracorporeal circuit, such as a dialysis circuit. In this embodiment, the pharmaceutical composition is added e.g. into the circuit at the dialyzer and administered to the subject through the extracorporeal circuit.

Suitable pharmaceutical compositions which are suitable for injection or infusion are known to the person skilled in the art. In this connection, for example, reference can be made in its entirety to K. H. Bauer et al., Lehrbuch der Pharmazeutischen Technologie [Textbook of Pharmaceutical Technology], WVG Stuttgart 1999. Pharmaceutical compositions which are suitable for injection are customarily sterile solutions, emulsions or suspensions, which are prepared by dissolving, emulsifying or suspending the active substance and optionally further excipients in water, in a suitable nonaqueous liquid which does not have to be sterile if this is justified, or in a mixture of these vehicles.

Pharmaceutical compositions which are suitable for infusion are customarily sterile, aqueous solutions or emulsions with water as the continuous phase.

Pharmaceutical compositions for injection or infusion can optionally contain further excipients. Excipients of this type are preferably solubilizers, substances for isotonicization, buffers, antioxidants, chelating agents, preservatives and emulsifiers.

Particularly preferred pharmaceutical compositions include locking solutions, catheter care and wound site infection gels and dialysates. A locking solution is a fluid used in a catheter or other desired patient access to the body. It contains a substance that is chosen for its ability to resist clotting, blockage and other impairments. A dialysate is a fluid used for fluid exchange in peritoneal dialysis. Diuresis refers to an osmotic gradient created for the purpose of limiting reabsorption of water.

In different embodiments of the invention, the dialysates preferably contain glucose, sodium chloride, calcium chloride, magnesium chloride, glucose and/or the conjugate L-P in at least about the millimolar to nanomolar range such as 1 x 10⁻³ to 1 x 10⁻⁹ mol/l and at least about 2 x 10⁻³ to 2 x 10⁻¹¹ mo/I and concentrations in between. It is important to note that glucose concentration may vary with an individual patient's needs. The conjugate L-P is preferably supplied in lyophilized sterile vials and is added to the fluid through standard aseptic techniques such as re-suspension of the lyophilized peptide and with sterile water, withdrawing the mixture into an appropriate size syringe and injecting the mixture into the fluid.

For purposes of preservation, the conjugate L-P may be kept dry until the time of use. It is also contemplated that the dialysate may contain the conjugate L-P in solution ready for peritoneal dialysis.

To meet the ultra filtration requirements of patients on peritoneal dialysis, the peritoneal dialysate is typically administered hyperosmolar relative to plasma to create an osmotic gradient that favors net movement of water into the peritoneal cavity; this is the nature of the ability of peritoneal dialysis to accomplish cleansing. In commercially available peritoneal dialysates, the sugar glucose serves as the osmotic agent that enhances ultra filtration. Available concentrations range from 1.5 wt.-% to 4.25 wt.-% dextrose or glucose.

In one embodiment of the invention, the conjugate L-P may be used as an adjunct to commercially available dialysates wherein the conjugate L-P may be added to the dialysate.

Locking solutions are also commercially available. "Heparin locks" are the most common locking solution in catheters in general and heparin is a common component of locking solutions in dialysis. In another embodiment of the invention, a locking solution containing anti-microbial and anti-inflammatory peptides is used for both peritoneal dialysis and hemodialysis. Parenteral anti-coagulants such as heparin, danaparoid and lepirudin are contemplated for use in the invention. A locking solution may contain the conjugate L-P preferably in an amount in the range of millimolar to nanomolar amounts.

For catheter care, the conjugate L-P can be mixed in a composition with a non-toxic, biologically compatible carrier prior to administration. Usually, this will be an aqueous solution, such as normal saline or phosphate-buffered saline (PBS), Ringer's solution, Ringer's lactate or any isotonic physiologically acceptable solution for administration by the chosen means. Preferably, the solution is manufactured and packaged under current Good Manufacturing Processes (GMP's) as approved by the FDA.

In another preferred embodiment, the pharmaceutical composition is formulated for oral administration. Preferably, the pharmaceutical composition is an oral administration form selected from the group consisting of tablets, powders, pellets, granules, sugar-coated tablets, syrups, juices, solutions, effervescent powders, effervescent granules, effervescent tablets, lyophilizates and capsules.

Suitable administration forms which are suitable for oral administration (oral medicaments) are known to the person skilled in the art. In this connection, reference can be made in its entirety, for example, to K. H. Bauer et al., Lehrbuch der Pharmazeutischen Technologie [Textbook of Pharmaceutical Technology], WVG Stuttgart 1999. Suitable excipients for the formulation of oral administration forms are known to the person skilled in the art. In this connection, reference can be made, for example, to H. P. Fiedler, Lexikon der Hilfstoffe für Pharmazie, Kosmetik und angrenzende Gebiete [Encyclopedia of excipients for pharmacy, cosmetics and related areas], Editio Cantor Aulendorf, 2001.

The conjugate L-P according to the invention is useful for dialysis such as hemodialysis and peritoneal dialysis, hemofiltration, plasmapheresis, apheresis, extracorporeal membrane oxygenation or assisted blood circulation.

In a preferred embodiment, the conjugate L-P is used to prevent and/or suppress inflammation, preferably in patients in the course of dialysis, hemofiltration, plasmapheresis, apheresis, extracorporeal membrane oxygenation or assisted blood circulation, preferably in patients suffering from ESRD.

A further aspect of the invention relates to the use of a conjugate L-P as described above for the manufacture of a pharmaceutical composition as described above
- for preventing and/or suppressing inflammation in dialysis patients;
- for preventing and/or treating end stage renal disease (ESRD);
- for preventing and/or treating autoimmune disease;
- for preventing and/or treating sepsis;
- for preventing and/or treating fever in dialysis patients; and/or
- for preventing and/or treating complications after transplantation.

The following examples further illustrate the invention but are not to be construed as limiting its scope.

### Example 1:

*Ex vivo* stimulation of human peripheral blood mononuclear cells (PBMCs)

PBMCs from healthy male volunteers (n=5) were isolated and stimulated as indicated below to mimic an uremic and inflammatory setting comparable to patients (LPS pre-incubation for 3 hours)

### Material and Methods

For the stimulation of PBMCs the following concentrations were chosen:
- LPS 1 ng/ml alone
- α-MSH
   ∘ 1µM; 10µM; 100µM
- Peg-KPV (pegylated tripeptide)
   ∘ 1µM; 10µM; 100µM; 1000µM

The stimulation procedure was as described below:

| Pre-incubation LPS | Co-stimulation LPS+Peptides |
|---|---|
| yes 3h | 24h |
| no- | 24h |

All cells were incubated for 24h and centrifuged at 1700 rpm and supernatants were used for TNF- α ELISA measurement according to manufacturer's protocols.

### Results

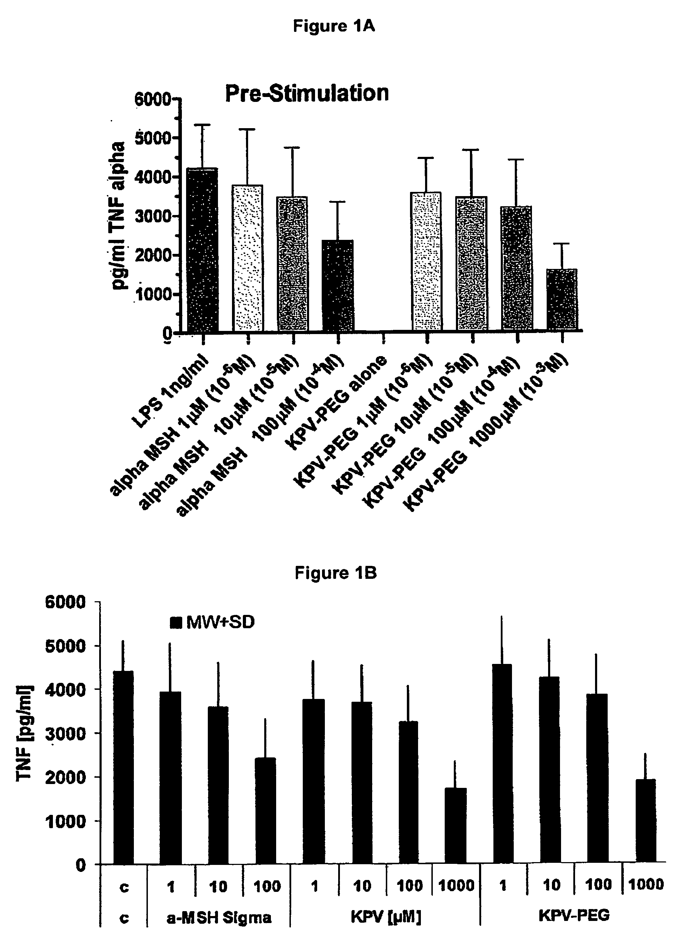

The results of the pre-stimulation are summarized in the following table and are additionally depicted in Figures 1A and 1 B:

| | TNF-α | LPS 3 h pre-incubation | | | | | | | |
|---|---|---|---|---|---|---|---|---|---|
| | | Donor 2 | Donor 3 | Donor 4 | Donor 5 | Donor 6 | MV+SD | SD | % |
| c | c | 4808 | 4690 | 4400 | 3157 | 4897 | 4390.4 | 714.521028 | 100.00 |
| α-MSH Sigma | 1 | 5005 | 4345 | 3968 | 2070 | 4286 | 3934.8 | 1108.54801 | 89.62 |
| | 10 | 4193 | 3883 | 3674 | 1799 | 4356 | 3581 | 1030.84262 | 81.56 |
| | 100 | 3009 | 2886 | 2073 | 942 | 2069 | 2395.8 | 906.537754 | 54.57 |
| KPV [µM] | 1 | 4025 | 4554 | 3541 | 2301 | 4299 | 3744 | 889.739288 | 85.28 |
| | 10 | 4126 | 4387 | 3666 | 2209 | 3949 | 3667.4 | 856.500029 | 83.53 |
| | 100 | 3542 | 5022 | 2886 | 1931 | 3731 | 3222.4 | 833.825102 | 73.40 |
| | 1000 | not done | 1816 | 1494 | 1054 | 2482 | 1711.5 | 601.166366 | 38.98 |
| Peg-KPV | 1 | 4082 | 4092 | 6032 | 3241 | 5201 | 4529.5 | 1090.9071 | 103.17 |
| | 10 | 4129 | 4265 | 5243 | 2875 | 4599 | 4222.2 | 867.392184 | 96.17 |
| | 100 | 4185 | 3610 | 4993 | 2435 | 3859 | 3816.4 | 931.790642 | 86.93 |
| | 1000 | not done | 1647 | 2224 | 2467 | 2467 | 1869 | 596.454525 | 42.57 |

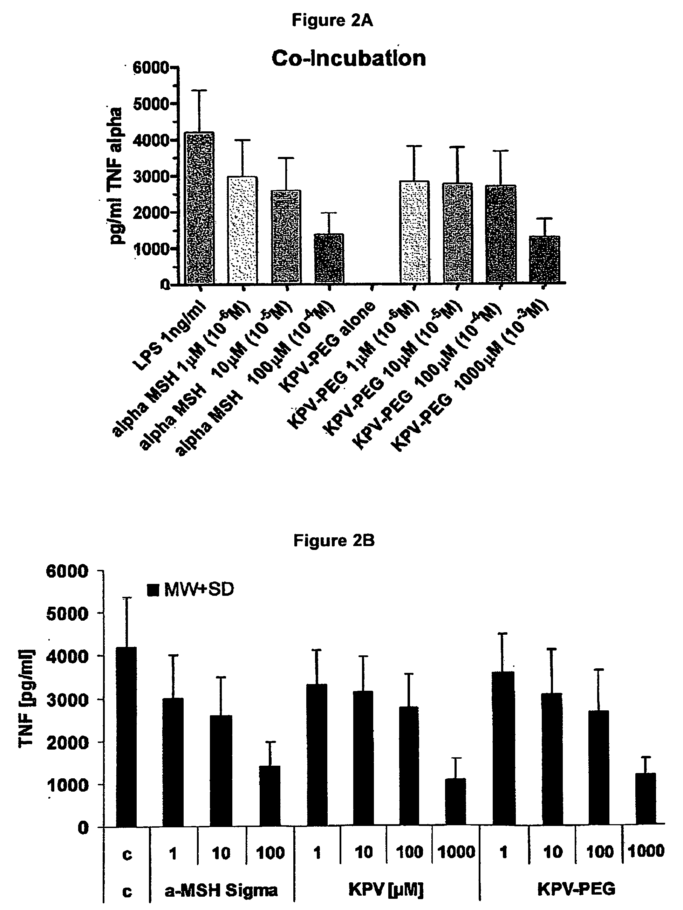

The results of the co-incubation are summarized in the following table and are additionally depicted in Figures 2A and 2B:

| | TNF-α | LPS + peptide simultaneously | | | | | | | |
|---|---|---|---|---|---|---|---|---|---|
| | | Donor 2 | Donor 3 | Donor 4 | Donor 5 | Donor 6 | MV+SD | SD | % |
| c | c | 4463 | 5268 | 4749 | 2207 | 4316 | 4200.6 | 1172.25181 | 100.00 |
| α-MSH Sigma | 1 | 4098 | 3273 | 3116 | 1319 | 3100 | 2981.2 | 1015.82267 | 70.97 |
| | 10 | 3734 | 2776 | 2568 | 1237 | 2638 | 2590.6 | 891.168783 | 61.67 |
| | 100 | 1207 | 2337 | 1248 | 713 | 1386 | 1380.2 | 593.368941 | 32.86 |
| KPV [µM] | 1 | 3426 | 3955 | 3702 | 1893 | 3541 | 3303.4 | 813.046309 | 78.64 |
| | 10 | 3154 | 3907 | 3369 | 1706 | 3470 | 3121.2 | 837.319154 | 74.30 |
| | 100 | 2562 | 3505 | 3077 | 1525 | 3142 | 2762.2 | 769.064822 | 65.76 |
| | 1000 | not done | 931 | 934 | 594 | 1801 | 1065 | 515.963177 | 25.35 |
| Peg-KPV | 1 | 3893 | 3697 | 3918 | 2021 | 4340 | 3573.8 | 899.137198 | 85.08 |
| | 10 | 3918 | 3274 | 2846 | 1317.5 | 3909 | 3052.9 | 1070.58526 | 72.68 |
| | 100 | 3617 | 3067 | 2305 | 1110.5 | 3088 | 2637.5 | 973.283874 | 62.79 |
| | 1000 | not done | 971 | 1428 | 694.5 | 1586 | 1169.875 | 410.414907 | 27.85 |

The data indicate an inhibitory effect for KPV and peg-KPV (provided by Invitrogen Life Technologies) as well as α-MSH for LPS-induced TNF-α release ("inflammation") when the inflammatory process has already started (pre-stimulation) or if the peptides and inflammatory stimulus is given simultaneously (co-incubation). The pre-incubation of LPS was used as a model to mimic a uremic situation in patients, in which inflammatory processes are ongoing already.

### Example 2:

Whole blood studies with blood from uremic patients including heat sterilization experiments

A first goal of this experiment was to test whether the three amino acid anti-inflammatory peptide KPV (unpegylated and pegylated version "monopeptide") will retain its activity under conditions of steam sterilization at 125°C for 15 min used in the production process of dialysis modules. A second goal was to study the anti-inflammatory effect of alpha-MSH and the peptides in uremic patients, which represent the real clinical situation.

Since peptides tend to lose activity when heated the experiment was designed to mimic the dialysis production process with the known parameters.

To perform this experiment whole blood samples were taken from voluntary uremic patients (n=10) and subsequently stimulated ex vivo with lipopolysaccharide (LPS) with or without alpha-MSH, KPV or pegylated KPV (non heated and heated) and TNF-α and IL-6 release was determined by ELISA.

### Material and Methods

25 ml blood from 10 uremic patients (dialysis patients treated at the hospital in Konstanz, Germany) was drawn and blood values were analyzed immediately. Blood was 1:10 diluted with PBS buffer and each stimulation condition was performed in triplicates and incubated at 200 µl in 96 well plates.

The following conditions were chosen:
- Unstimulated blood
- Blood stimulated with LPS 1 ng/ml alone (maximal stimulation of pro-inflammatory cytokine TNF-α.
- Blood stimulated with LPS 1 ng/ml and 4 different concentrations of α-MSH
   ∘ 0.1µM; 1µM; 10 µM; 100µM
- Blood stimulated with LPS 1 ng/ml and 5 different concentrations of
   o KPV (0.1 µM; 1 µM; 10pM; 100µM; 1000µM)
   o KPV-peg (0.1µM; 1µM; 10µM; 100µM; 1000µM)
- Blood stimulated with LPS 1 ng/ml and 5 different concentrations of
   o KPV heated(0.1µM; 1µM; 10µM; 100µM; 1000µM)
   o KPV-peg heated (0.1µM; 1µM; 10µM; 100µM; 1000µM)

Blood was incubated for 24 h, centrifuged at 1700 rpm and serum supernatants were used for TNF-α and IL-6 ELISA measurement according to manufacturer's protocols.

### Results

TNF-α and IL-6 ELISA showed that the heat treatment did not influence the activity of the peptide. The average inhibition for the tested concentrations of KPV for TNF-α was around 30% and for and IL-6 around 20%.

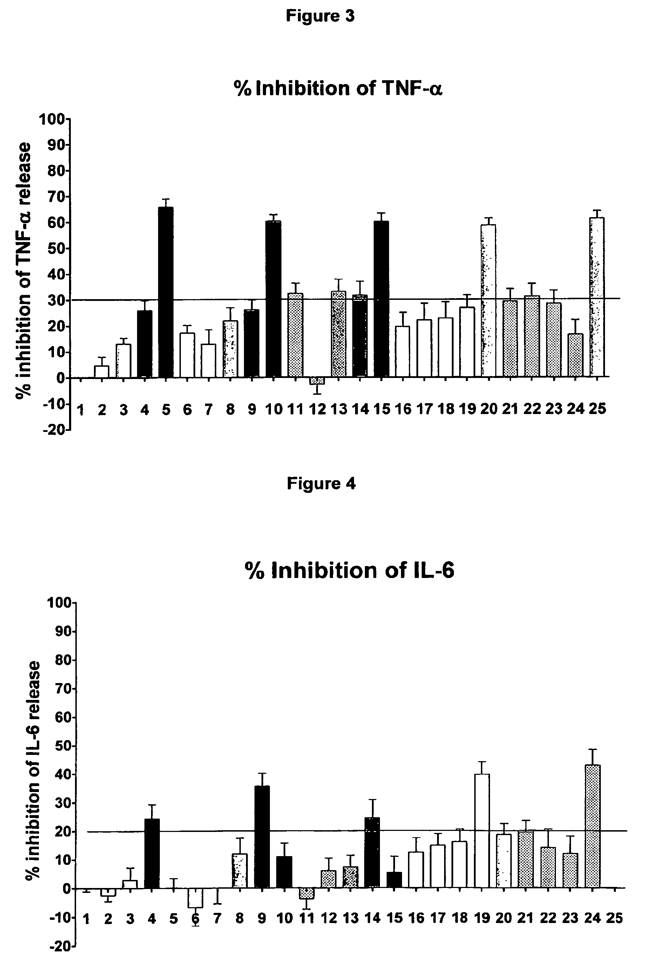

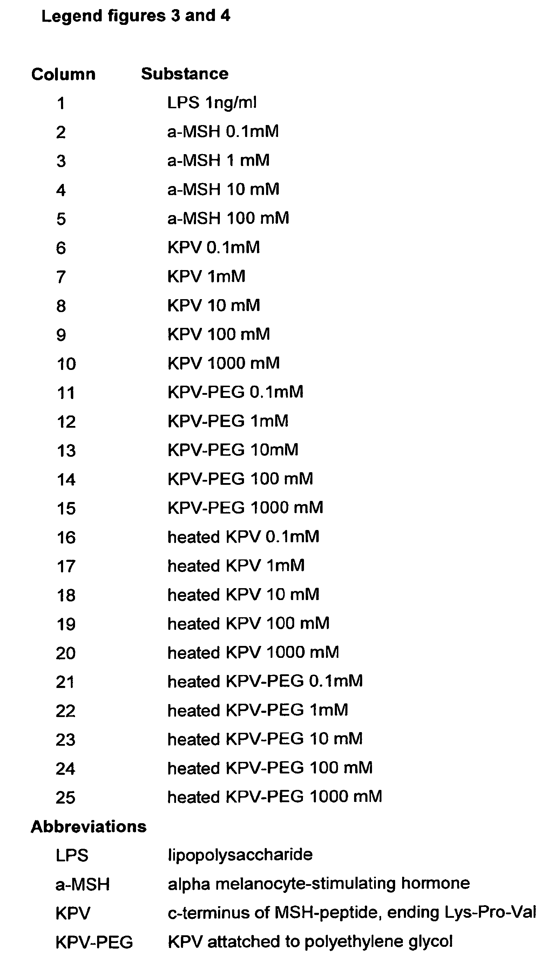

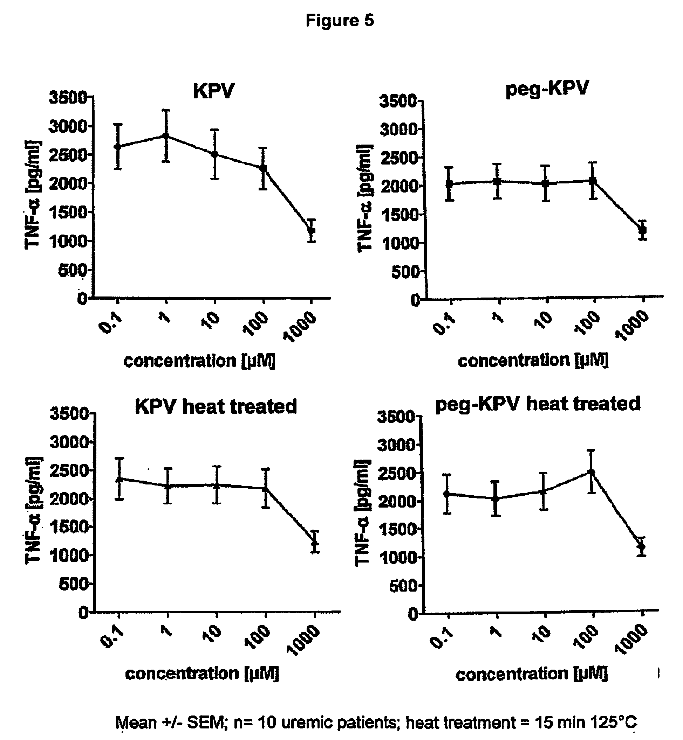

The results are further depicted in Figures 3, 4 and 5.

## Claims

1. A conjugate L-P according to general formula (VI): 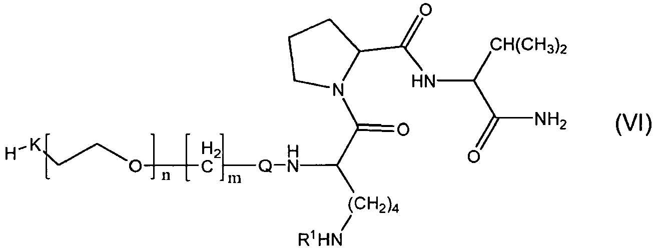 wherein
R¹ is -H or -C(=O)-C₁₋₈-aliphate;
Q is a bond or a functional group selected from -C(=O)-, -C(=S)-, -C(=NH)-, -NH-C(=O)-, -NH-C(=S)- and -NH-C(=NH)-;
K is a bond or a functional group selected from -C₁₋₈-alkylene-, -NH-, -O-, -S-, -C(=O)-, or -C(=O)O-;
n is an integer of from 0 to 250; and
m is an integer of from 0 to 12;
or according to general formula (VII): 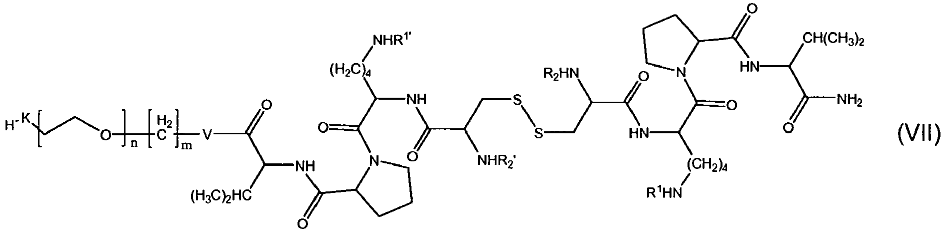 wherein
R¹, R¹', R² and R²' are independently of each other -H or -C(=O)-C₁₋₈-aliphate;
V is a bond or a functional group selected from -O- and -NH-;
K is a bond or a functional group selected from -C₁₋₈-alkylene-, -NH-, -O-, -S-, -C(=O)-, or -C(=O)O-;
n is an integer of from 0 to 250; and
m is an integer of from 0 to 12.

2. A conjugate L-P as defined in claim 1 for use in prevention and/or suppression of inflammation in dialysis patients.

3. A conjugate L-P as defined in claim 1 for use in prevention and/or suppression of inflammation in dialysis patients, wherein the patients suffer from end stage renal disease (ESRD).

4. A conjugate according to claim 1 for use in hemodialysis, hemofiltration, plasmapheresis, apheresis, extracorporeal membrane oxygenation or assisted blood circulation.

5. Use of a conjugate L-P according to claim 1 as an intermediate in the preparation of a construct S-L-P by attaching the conjugate L-P to a surface S of a solid support.

## Patentansprüche

1. Konjugat L-P nach der allgemeinen Formel (VI): 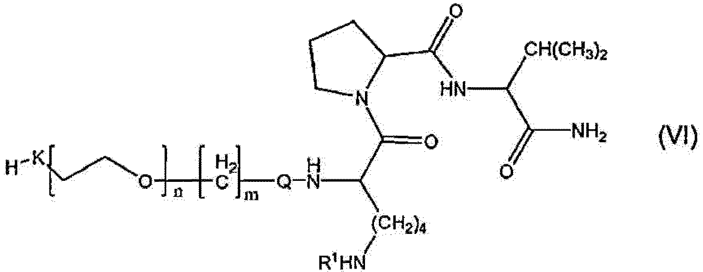 wobei
R¹ -H oder -C(=O)-C₁₋₈-Aliphat ist;
Q eine Bindung oder eine funktionelle Gruppe, ausgewählt aus -C(=O)-, -C(=S)-, -C(=NH)-, -NH-C(=O)-, -NH-C(=S)- und -NH-C(=NH)- ist;
K eine Bindung oder eine funktionelle Gruppe, ausgewählt aus -C₁₋₈-Alkylen-, -NH-, -O-, -S-, -C(=O)-oder -C(=O)O- ist;
n eine ganze Zahl von 0 bis 250 ist; und
m eine ganze Zahl von 0 bis 12 ist;
oder nach der allgemeinen Formel (VII): 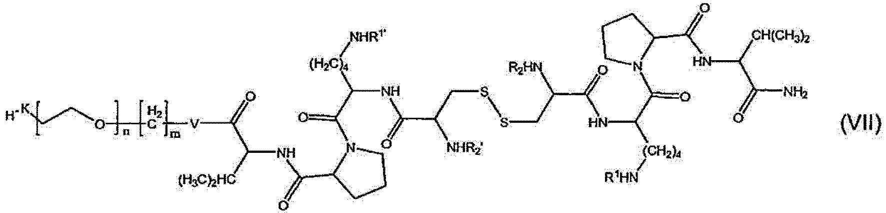 wobei
R¹, R¹', R² und R^{2'} unabhängig voneinander -H oder-C(=O)-C₁₋₈-Aliphat sind;
V eine Bindung oder eine funktionelle Gruppe, ausgewählt aus -O- und -NH- ist;
K eine Bindung oder eine funktionelle Gruppe, ausgewählt aus -C₁₋₈-Alkylen-, -NH-, -O-, -S-, -C(=O)-oder -C(=O)O- ist;
n eine ganze Zahl von 0 bis 250 ist; und
m eine ganze Zahl von 0 bis 12 ist.

2. Konjugat L-P nach Anspruch 1 zur Verwendung in der Vorbeugung und/oder Unterdrückung einer Entzündung bei Dialysepatienten.

3. Konjugat L-P nach Anspruch 1 zur Verwendung in der Vorbeugung und/oder Unterdrückung einer Entzündung bei Dialysepatienten, wobei die Patienten an einer Nierenerkrankung im Endstadium (End Stage Renal Disease, ESRD) leiden.

4. Konjugat L-P nach Anspruch 1 zur Verwendung in Hämodialyse, Hämofiltration, Plasmapherese, Apherese, extrakorporealer Membranoxygenierung oder unterstützter Blutzirkulation.

5. Konjugat L-P nach Anspruch 1 als Zwischenprodukt in der Zubereitung eines Konstrukts S-L-P durch Anheften des Konjugats L-P an eine Oberfläche S eines festen Trägers.

## Revendications

1. Conjugué L-P selon la formule générale (VI) : 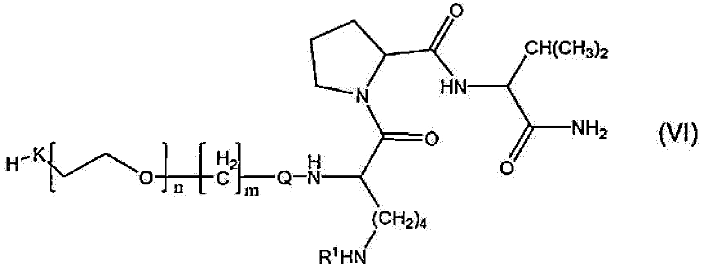 dans laquelle
R1 est du -H ou -C(=O)-C₁₋₈-aliphate ;
Q est une liaison ou un groupe fonctionnel sélectionné parmi -C(=O)-, -C(=S)-, -C(=NH)-, -NH-C(O)-, -NH-C(=S) et -NH-C(=NH)- ;
K est une liaison ou un groupe fonctionnel sélectionné parmi -C₁₋₈-alkylène-, -NH-, -O-, -S- -C(=O) ou-C(=O)O- ;
n est un nombre entier allant de 0 à 250 ; et
m est un nombre entier allant de 0 à 12 ;
ou selon la formule générale (VII) : 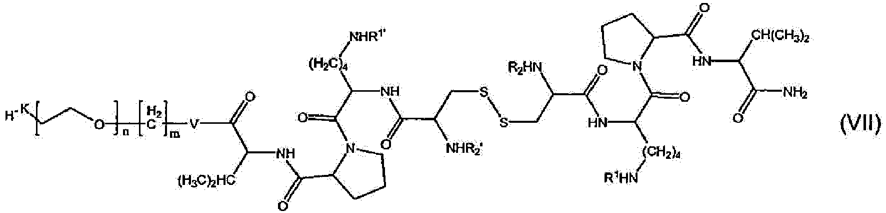 dans laquelle
R1, R1', R2 et R2' sont indépendamment l'un de l'autre du -H ou -C(=O)-C₁₋₈-aliphate ;
V est une liaison ou un groupe fonctionnel sélectionné parmi -O- et-NH-;
K est une liaison ou un groupe fonctionnel sélectionné parmi -C₁₋₈-alkylène-, -NH-, -O-, -S- -C(=O)-, ou - C(=O)O- ;
n est un nombre entier allant de 0 à 250 ; et
m est un nombre entier allant de 0 à 12.

2. Conjugué L-P selon la revendication 1 destiné à un usage dans la prévention et/ou l'élimination de l'inflammation chez des patients sous dialyse.

3. L-P selon la revendication 1 destiné à un usage dans la prévention et/ou l'élimination de l'inflammation chez des patients sous dialyse, les patients souffrant d'une maladie rénale au stade terminal (ESRD).

4. Conjugué selon la revendication 1 destiné à un usage en hémodialyse, hémofiltration, plasmaphérèse, aphérèse, oxygénation de membrane extracorporelle ou circulation sanguine assistée.

5. Utilisation du conjugué L-P selon la revendication 1 comme intermédiaire dans la préparation d'une structure S-L-P en fixant le conjugué L-P à une surface S d'un support solide.
